# EUROPEAN PATENT APPLICATION

(11) **EP 4 403 238 A1**
(43) Date of publication of application: **24.07.2024**
(21) Application number: 23184784.9
(22) Date of filing: 11.07.2023
(51) Int. Cl.: B01D 3/14, B01D 53/02, C02F 1/28, C07C 41/36, C07D 301/12

(54) **PROCESS AND APPARATUS FOR RECOVERING METHOXYPROPANOLS FROM AN AQUEOUS STREAM**

(71) Applicant: ThyssenKrupp Uhde GmbH, 44141 Dortmund (DE); thyssenkrupp AG, 45143 Essen (DE)
(72) Inventor: Benje, Michael, 65812 Bad Soden (DE); Kleiber, Michael, 65795 Hattersheim (DE); Dietz, Hans-Christian, 65795 Hattersheim (DE)
(74) Representative: thyssenkrupp Intellectual Property GmbH

(57) **Abstract**

The invention relates to a process for recovering methoxypropanols (24) from an aqueous stream (16, 27), wherein the process comprises the steps of obtaining a purified aqueous stream (21) by passing the aqueous stream (16, 27) through an adsorption unit (28) containing an adsorption agent for adsorbing the methoxypropanols to the adsorption agent, desorbing the methoxypropanols from the adsorption agent, wherein the step of desorbing the methoxypropanols comprises passing an alcohol as a desorption agent (30) through the adsorption unit (28) to desorb and discharge the methoxypropanols (24) from the adsorption agent, separating the methoxypropanols (24) from the desorption agent (30) by distillation, an apparatus for recovering methoxypropanols using the aforementioned process and a plant for producing of propylene oxide comprising such an apparatus.

## Description

### Field of the invention

The invention is directed to a process and an apparatus for recovering 1-methoxy-2-propanol and 2-methoxy-1-propanol from an aqueous stream, in particular from an effluent stream formed in a process for making propylene oxide by epoxidation of propylene with hydrogen peroxide in an organic solvent, preferably methanol.

### Background of the invention

The epoxidation of propylene with hydrogen peroxide in a methanol solvent, typically in the presence of a titanium zeolite epoxidation catalyst, has become a major industrial process for manufacturing propylene oxide. Since water is formed from hydrogen peroxide in the reaction and hydrogen peroxide is used as an aqueous solution, this process produces an aqueous effluent stream. Besides other by-products, which occur in smaller amounts, this aqueous effluent stream contains dissolved 1-methoxy-2-propanol and 2-methoxy-1-propanol formed as by-products from the reaction of propylene oxide with solvent methanol as main components.

Recovering the methoxypropanols from the aqueous effluent stream is desirable not only because they have commercial value, but also for reducing the TOC (total organic carbon) of the aqueous effluent stream. By reducing the TOC of the aqueous effluent stream, it is possible to reduce the size and thereby the investment cost of a downstream biological wastewater treatment unit. Furthermore, removal of the methoxypropanols significantly lowers the operating cost of the biological wastewater treatment unit, which significantly contributes to the operating cost of a propylene oxide production process.

In the literature, several processes and facilities for removing by-products of the propylene epoxidation from an aqueous stream are described. A major part of this bibliography discloses processes and devices based on extractive removal of such by-products from the water stream. As an example, representative for extractive processes, the process according to WO 2021/204531 A1 is referred to. This process is directed to the recovery of methoxypropanols from an aqueous stream.

According to WO 2021/204531 A1, the aqueous stream is treated in an extraction column with an extracting agent in countercurrent. The extract stream is then separated by an extractive distillation, giving a bottoms stream, which is depleted of water content relative to the feed extract stream. This bottom stream is then distilled, drawing off a mixture of 1-methoxypropanol and 2-methoxypropanol overheads and recovered extracting agent as bottoms stream.

Due to the usage of extraction equipment, especially extraction columns, those processes are technically complex. Furthermore, a certain amount of the extracting agent remains in the treated water stream and causes the need for an additional separation step for its removal.

In order to overcome the drawbacks of an extraction-based recovery process of methoxypropanols, several adsorption/desorption processes in the treatment of water streams have already been described in the patent literature:
US 5,999,955 A describes the treatment of a water stream from an epoxidation zone for the production of propylene oxide in order to remove heavy components like propyleneglycols and methoxypropanols. According to US 5,999,955 A, the water phase is treated in a separation zone, such as an evaporator, a distillation zone or a sorption zone. From the sorption zone, a mixture of heavy organic components is recovered by desorption. The heavy organ-ics stream can be used as a solvent or can be further processed for the recovery of propylene glycol.

However, US 5,999,955 A does not disclose how the desorption of heavy components is effected and which desorption mechanism is to be used. Furthermore, US 5,999,955 A does not disclose a process for the recovery of methoxypropanols from the wastewater or the heavy components stream.

US 2016/0130160 A1 discloses a process for reducing the total organic carbon in wastewater from a process for the production of propylene oxide by combining an adsorbing agent and a reverse osmosis membrane. According to US 2016/0130160 A1, the waste water first passes an adsorbing agent, where a part of the contained oxygenates is adsorbed and after that passes a reverse osmosis membrane, where the water stream is furtherly depleted of oxygenates. In the process disclosed by US 2016/0130160 A1, the adsorbing agent mainly serves to protect of the reverse osmosis membrane from being deactivated by anthraquinone or anthraquinone derivatives from an upstream plant for the production of hydrogen peroxide, which is used as an oxidation agent in the process for the production of propylene oxide. US 2016/0130160 A1 furtherly discloses that all oxygenates can be adsorbed by the adsorbing agent for a certain time in case that the reverse osmosis membrane is spent and needs to be replaced or for the time that is needed to direct the waste water stream from a spent reverse osmosis membrane unit to a fresh one.

However, US 2016/0130160 A1 does not disclose a process for the desorption or recovery of adsorbed compounds from the adsorbing agent. Furthermore, US 2016/0130160 A1 does not disclose a process for the recovery of methoxypropanols from the wastewater stream.

### Brief Summary of Invention

The object of the invention therefore is to provide a process and an apparatus for recovering methoxypropanols from an aqueous stream that effectively reduces the TOC of the aqueous stream, makes available the methoxypropanols for further use and is easy to integrate in a plant for producing propylene oxide.

This object is achieved by the process for recovering methoxypropanols from an aqueous stream according to the features of claim 1.

Hereby, a process for recovering methoxypropanols from an aqueous stream is provided, which comprises the steps of obtaining a purified aqueous stream by passing the aqueous stream through an adsorption unit containing an adsorption agent for adsorbing the methoxypropanols to the adsorption agent and afterwards desorbing the methoxypropanols from the adsorption agent. According to the invention, the step of desorbing the methoxypropanols comprises passing an alcohol as a desorption agent through the adsorption unit to desorb and discharge the methoxypropanols from the adsorption agent and separating the methoxypropanols from the desorption agent by distillation.

According to the invention, the adsorption agent is regenerated by way of solvent desorption, using an alcohol as the desorption agent. As compared to purely thermal desorption, solvent desorption has the advantage that it results in a very efficient and effective desorption process in a rather short period of time and, moreover, allows the adsorption agent to stay within the adsorption unit during regeneration. The use of a liquid desorption agent for solvent desorption therefore has major advantages in a setup, in which large amounts of adsorption agent are to be regenerated on a regular basis. Further, using an alcohol as the desorption agent for desorbing methoxypropanols has the advantage that this combination is particularly well suited for a downstream separation by distillation, as the boiling points show a sufficient difference, and no azeotrope is formed. By means of distillation, both components can be put to a further use: The methoxypropanols may be used as a product of value and the alcohol may be reused as desorption agent. Recycling the desorption agent has the advantage that the distillation may be optimized to recover the methoxypropanols at a high purity, leaving remnants of methoxypropanols in the desorption agent, that do not harm in a further desorption process.

In advantageous embodiments, a polyhydric alcohol, preferably propylene glycol is used as the desorption agent. Polyhydric alcohols show a particularly high selectivity for desorbing methoxypropanols and thus improve the desorption process. In particular, propylene glycol is a polyhydric alcohol that is typically produced as a by-product in plants for producing of propylene oxide, and thus can readily be used when integrating the process in a propylene oxide production plant.

Preferably, the desorption is carried out at an elevated temperature compared to ambient temperature. The temperature of the desorption agent passed through the adsorption unit may preferably be at least 60 °C, and even more preferred, at least 70 °C. Desorption at elevated temperatures increases the efficiency of the desorption process, leading to reduced desorption times.

In advantageous embodiments, prior to passing the aqueous stream through the adsorption unit, methanol is removed from the aqueous stream by distillation. Methanol is a contaminant that is typically present in effluent aqueous streams of propylene oxide production plants. When applying the process according to the invention it is preferred to remove the methanol prior to the adsorption unit, because methanol in the feed stream to the adsorption competes with methoxypropanols in regard to adsorption capacitiy and may cause problems in the methoxypropanol purification step. Furthermore it can be removed from the feed to the adsorption rather easily by way of distillation.

Preferably, before the desorbing step is started, the adsorption unit is flushed with the desorption agent to displace remaining water in the adsorption unit resulting in a desorption agent/water-mixture forming a first flushing effluent. Flushing the adsorption unit before starting the desorption and discharging the first flushing effluent protects the desorption agent cycle from the introduction of and contamination with water.

Further preferably, after the desorbing step, the adsorption unit is flushed with the aqueous stream to displace remaining desorption agent in the adsorption unit resulting in a desorption agent/water-mixture forming a second flushing effluent. Flushing the adsorption unit after the end of the desorption and discharging the second flushing effluent protects the purified aqueous stream from being contaminated by remaining desorption agent, when the adsorption process for the aqueous stream is started once again.

In preferred embodiments, the adsorption unit is divided in at least two separately operable sections, whereof at least temporarily the first section is operated in an adsorption mode, while a second section is operated in a desorption mode at the same time. Having two sections of the adsorption unit that are separately operable in this way allows the adsorption unit to be operated continuously in adsorption mode with at least one of the sections, and thus being continuously able to treat an aqueous input stream. Desorption of the individual sections of the adsorption unit can thus be carried out on-line, without the need to stop the aqueous input stream in the meantime.

The object is further solved by a process for producing propylene oxide, comprising the steps of
- catalytically oxidizing propylene with an aqueous hydrogen peroxide solution using methanol as solvent thereby obtaining a reaction mixture,
- separating from the reaction mixture
   - unreacted propylene in a propylene separation unit to be recycled,
   - propylene oxide as main reaction product in a propylene oxide separation unit,
   - methanol as the solvent in a methanol separation unit to be recycled, and
   - propylene glycol as a reaction by-product in a propylene glycol separation unit,
- leaving a remaining aqueous stream containing methoxypropanols.
According to the invention, the methoxypropanols are recovered from the aqueous stream using the process as described before.

Preferably, the propylene glycol obtained as reaction by-product is used as the desorption agent. This has the advantage that the reaction by-product propylene glycol is put to direct use within the process for producing propylene oxide and the recovery of methoxypropanols can be integrated more easily into the process for producing propylene oxide.

In particular, it is preferred, if the first flushing effluent and/or the second flushing effluent is recycled to the propylene glycol separation unit. In the combination of the use of propylene glycol as the desorption agent and recycling the flushing effluents to the propylene glycol separation unit, the advantages of flushing the adsorption unit prior and/or after desorption can be accomplished without the need for separate equipment for treating the flushing effluents. As the flushing effluents mainly comprise a mixture of water and desorption agent, i.e. in this case propylene glycol, the propylene glycol separation unit that is needed in the process for producing propylene oxide, anyhow, can be used for treating the flushing effluents, as well.

In terms of apparatus features, the object is achieved by an apparatus for recovering methoxypropanols from an aqueous stream. The apparatus comprises an adsorption unit comprising an adsorption agent for adsorbing the methoxypropanols and having an inlet and an outlet. According to the invention, the apparatus further comprises a buffer vessel for storing a desorption agent, a distillation unit for separating the methoxypropanols from the desorption agent, and first and second switching means. The buffer vessel and a source of the aqueous stream are connected to the inlet via the first switching means and the distillation unit and a sink for a purified aqueous stream are connected to the outlet via the second switching means, for alternately passing the aqueous stream and the desorption agent through the adsorption unit to switch over between in an adsorption mode and a desorption mode of the adsorption unit. The distillation unit is connected to the buffer vessel for recycling the desorption agent.

Preferably, between the source of the aqueous stream and the first switching means a methanol stripping column is arranged in order to remove methanol from the aqueous stream by distillation.

Finally, the object is achieved by a plant for producing of propylene oxide. The plant comprises a reactor section for catalytically oxidizing propylene with an aqueous hydrogen peroxide solution using methanol as solvent thereby obtaining a reaction mixture. Downstream of the reactor section the plant further comprises a separation section for obtaining an aqueous stream from the reaction mixture. The separation section comprises a propylene separation unit for separating unreacted propylene from the reaction mixture to be recycled to the reactor section, a propylene oxide separation unit for separating and purifying propylene oxide from the reaction mixture as main reaction product, a methanol separation unit for separating methanol from the reaction mixture to be recycled to the reactor section, and a propylene glycol separation unit for separating propylene glycol from the reaction mixture as a reaction by-product. According to the invention, the plant comprises downstream of the separation section an apparatus for recovering methoxypropanols from the aqueous stream obtained by the separation section as described above.

Preferably, in the plant for producing of propylene oxide the propylene glycol separation unit is connected to the buffer vessel in order to provide the reaction by-product propylene glycol as the desorption agent. By this connection, propylene glycol can be initially provided to the propylene glycol cycle and the propylene glycol cycle may be topped-up for any propylene glycol leaving the cycle, e.g. during flushing of the adsorption unit.

In preferred embodiments, the second switching means is connected to the propylene glycol separation unit by means of a connecting line for recycling flushing effluents obtained in a flushing mode of the adsorption unit to the propylene glycol separation unit.

Preferably, the adsorption unit contains at least two sections that are connected to the first and second switching means in such a way, that a first section can be operated in an adsorption mode, while a second section is operated in a desorption mode at the same time. Thereby a continuous operation in the adsorption mode is facilitated, while the sections of the adsorption unit may be regenerated on-line individually by desorption, when needed.

Further advantages of the invention are described in the following with regard to the embodiments shown in the attached drawings.

### Brief Description of Drawings

- Fig. 1: shows schematically a plant for producing of propylene oxide according to the invention, which comprises an apparatus for recovering methoxypro-panols from an aqueous stream,
- Fig. 2: shows schematically a first embodiment of an apparatus for recovering 1-methoxypropanols and 2-methoxypropanols from an aqueous stream in a mixed methoxypropanol stream,
- Fig. 3: shows schematically a second embodiment of an apparatus for recovering methoxypropanols from an aqueous stream comprising additional devices to separate the recovered 1-methoxypropanol and 2-methoxypropanol fractions by distillation.

### Detailed Description of the Invention

In the drawings same parts are consistently identified by the same reference signs and are therefore generally described and referred to only once.

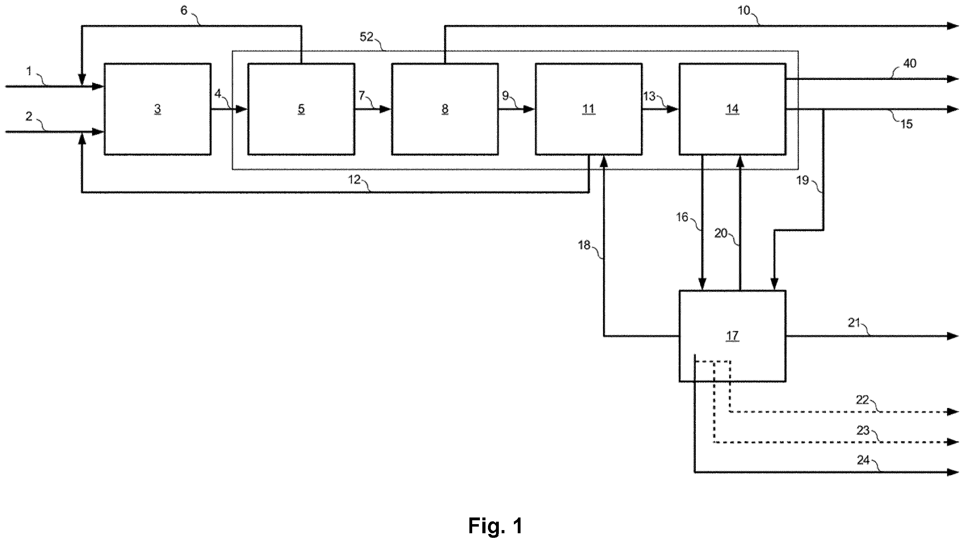

In Fig. 1, a plant for producing of propylene oxide, 10, is shown. In the plant, fresh propylene 1 and recycled propylene 6 are fed into a reactor section 3 for catalytically oxidizing the propylene 1, 6. The oxidization is carried out with an aqueous hydrogen peroxide solution 2 using (recycled) methanol 12 as solvent. In the reactor section 3, propylene 1, 6 is catalytically oxidized by hydrogen peroxide 2, forming propylene oxide and water as a co-product. A resulting reaction mixture 4 is obtained and discharged via an outlet of the reactor section 3.

The reaction mixture 4 typically contains propylene oxide, water, unconverted propylene, methanol and oxygenated by-products, among them monopropylene glycol (MPG), 1-methoxypropanol (1-MPol) and 2-methoxypropanol (2-MPol). Further oxygenated by-products are high boiling compounds like dipropylene glycol (DPG) and tripropylene glycol (TPG) and their methylethers.

Downstream of the reactor section 3 the plant comprises a separation section 52. The separation section is used to separate several components of the reaction mixture 4 and to obtain an aqueous stream 16 that is left over at the end of the separation section 52. In detail the separation section 52 comprises at least
- a propylene separation unit 5 for separating unreacted propylene 6 from the reaction mixture 4 to be recycled to the reactor section 3, leaving behind a reaction mixture depleted by propylene 7,
- a propylene oxide separation unit 8 for separating propylene oxide 10 from the reaction mixture 7 as main reaction product of the plant, leaving behind a reaction mixture depleted by propylene oxide, 9,
- a methanol separation unit 11 for separating methanol 12 from the reaction mixture 9 to be recycled to the reactor section 3 as solvent, leaving behind a reaction mixture depleted by methanol 13, and
- a propylene glycol separation unit 14 for separating propylene glycol 15 from the reaction mixture 13 as a reaction by-product and other high boiling components 40, leaving behind an aqueous stream 16.

After having passed the methanol separation unit 11, the reaction mixture depleted by methanol, 13, contains propylene glycols (MPG, DPG, TPG) and their methylethers, as well as other high boiling organic components and dissolved salts. Furthermore, the reaction mixture depleted by methanol 13 contains methoxypropanols, in particular 1-methoxypropanol and 2-methoxypropanol.

The reaction mixture depleted by methanol 13 is fed to the propylene glycol (PG) separation unit 14, in which propylene glycol 15 is separated. The propylene glycol separation unit 14 may further comprise a purification unit, in which propylene glycol 15 is purified, e.g. by evaporation and distillation, resulting in a purified propylene glycol stream 15 and a mixed stream 40. In this case, the mixed stream 40 comprises higher glycols and possibly other higher boiling compounds that are separated from the propylene glycol 15, which mainly comprises monopropylene glycol.

The aqueous stream 16 leaving the propylene glycol separation unit 14 is depleted by glycols and contains methoxypropanols and possibly methanol residues as main contaminants as well as traces of other organic components. Especially methanol and methoxypropanols need to be reduced before the aqueous stream 16 can be treated in a conventional waste-water treatment plant in order to reduce investment and operational cost for said waste-water treatment plant.

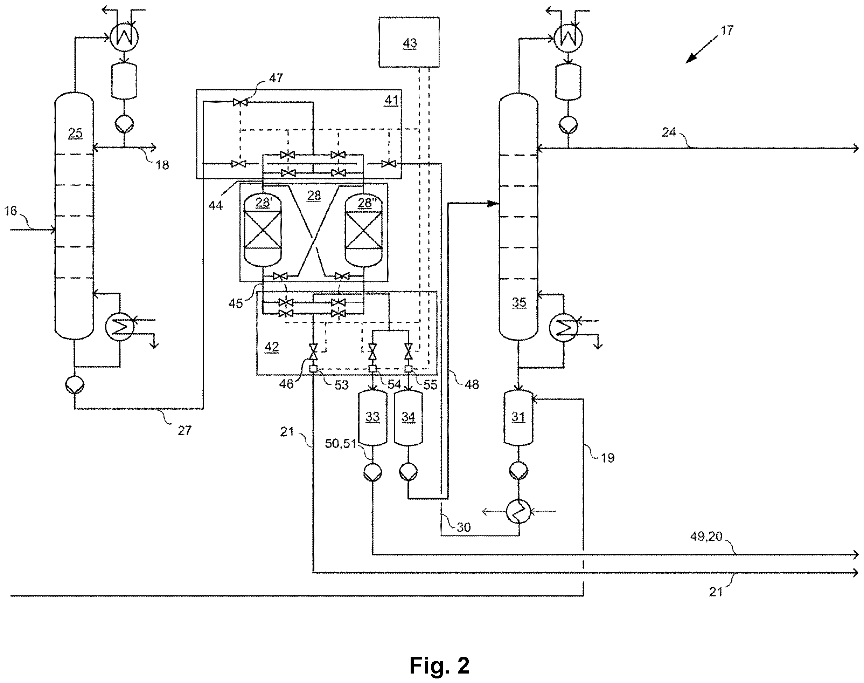

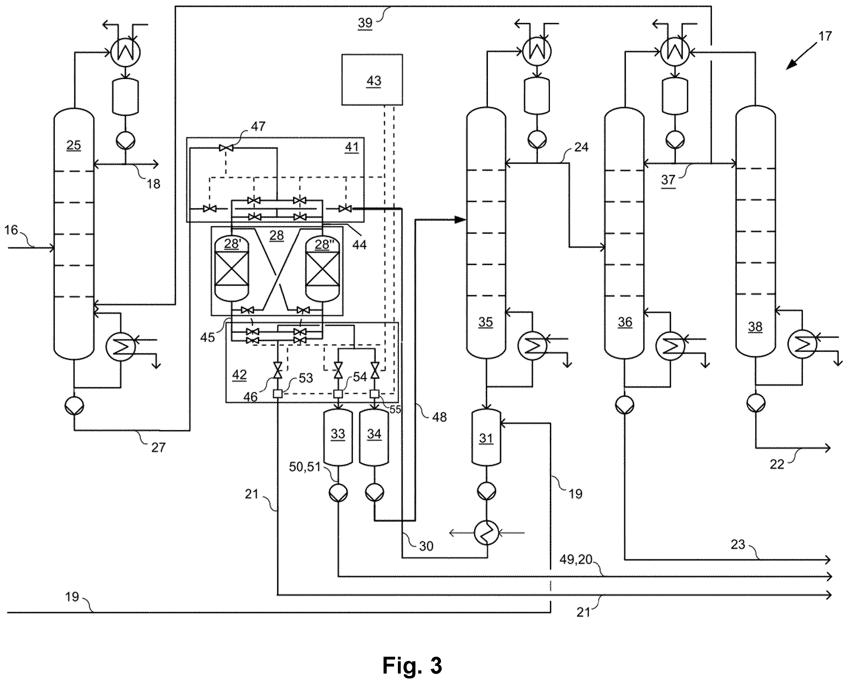

To this end, the plant for production of propylene oxide comprises an apparatus 17 for recovering methoxypropanols from the aqueous stream 16 according to the invention that will be described in more detail below with regard to Fig. 2 and 3.

As shown in Fig. 1, the apparatus 17 for recovering methoxypropanols is preferably integrated into the plant for producing propylene oxide as follows:
The apparatus 17 treats the aqueous stream 16 by means of an adsorption unit, resulting in a purified aqueous stream 21. The apparatus 17 optionally contains a methanol stripping column in order to recover remaining methanol 18 from the aqueous stream 16 by distillation. The recovered methanol 18 is then preferably fed back to the methanol separation unit 11 and/or recycled to the reactor section 3 as recycled methanol 12.

On a regular basis, the adsorption unit needs to be regenerated by means of desorption using a desorption agent. Preferably, the apparatus 17 makes use of the reaction by-product propylene glycol 15 as the desorption agent. To this end, it is advantageous to branch off at least a partial stream of propylene glycol 19 from the by-product propylene glycol 15 and feed it to the apparatus 17 as a source of desorption agent.

In order to make sure that water is not introduced into the desorption agent cycle and that remnants of desorption agent are not introduced into the purified aqueous stream 21, it is advantageous to flush the adsorption unit with desorption agent before desorption agent and after desorption flushing the adsorption unit with aqueous stream. The flushing effluents, being desorption agent/water mixtures, 20, are preferably returned to the propylene glycol separation unit 14.

From the desorption agent loaded with methoxypropanols the methoxypropanols are separated by distillation. Depending on the embodiment of the apparatus 17, the methoxypropanols may be discharged from the apparatus 17 as a stream of mixed methoxypropanols 24, or it may contain additional means for obtaining purified 1-methoxypropanol 22 and purified 2-methoxypropanol 23.

**Fig.** 2 shows a first embodiment of an apparatus for recovering methoxypropanols 24 from an aqueous stream 16, 27. The apparatus 17 comprises an adsorption unit 28 comprising an adsorption agent for adsorbing the methoxypropanols and having at least one inlet 44 and at least one outlet 45. The adsorption agent may for example be based on activated charcoal.

The apparatus 17 further comprises a buffer vessel 31 for storing a desorption agent 30 and a distillation unit 35 for separating the methoxypropanols 24 from the desorption agent 30. The distillation unit 35 is connected to the buffer vessel 31 for recycling the desorption agent 30.

Also, the apparatus 17 comprises first and second switching means 41, 42. The desorption agent buffer vessel 31 and a source of the aqueous stream, 16, 27, are connected to the inlet 44 of the adsorption unit 28 via the first switching means 41. The distillation unit 35 and a sink for a purified aqueous stream 21 are connected to the outlet 45 of the adsorption unit 28 via the second switching means 42. The switching means 41, 42 are configured for alternately passing the aqueous stream 16, 27 and the desorption agent 30 through the adsorption unit 28 to switch over between in an adsorption mode and a desorption mode of the adsorption unit 28.

Within the plant for producing propylene oxide shown in Fig. 1, the propylene glycol separation unit 14 forms the source of the aqueous stream 16. Preferably, the buffer vessel 31 is connected to the propylene glycol separation unit 14 in order to provide the reaction by-product propylene glycol 19 as make-up stream to the desorption agent 30. Further preferably, the second switching means 42 is connected to the propylene glycol separation unit 14 by means of a connecting line for recycling flushing effluents 49 obtained in a flushing mode of the adsorption unit 28 to the propylene glycol separation unit 14.

In advantageous embodiments as shown in Fig. 2, the adsorption unit 28 contains at least two sections 28', 28", that are individually connected to the first 41 and second switching means 42 in such a way, that a first section 28' can be operated in an adsorption mode, while a second section 28"is operated in a desorption mode at the same time. The first 41 and second switching means 42 may comprise controllable valves 47, 46 that are actuated by a control unit 43. Preferably, the first switching means 41 is configured such that either of the input streams, the aqueous stream 16, 27 and the desorption agent 30, can be fed into any one of the sections 28', 28" of the adsorption unit 28, independently. The second switching means 42 is preferably configured such that the effluent stream of each of the sections 28', 28" of the adsorption unit 28 can be discharged either to the distillation column 35, to the purified aqueous stream 21 or - as a flushing effluent 50, 51 - to the connecting line for recycling flushing effluents 49.

In the preferred embodiment shown in Fig. 2, in the apparatus 17 a methanol stripping column 25 is arranged between the source of the aqueous stream 16 and the first switching means 41 in order to remove remaining methanol 18 from the aqueous stream 16 by distillation.

When in operation, the apparatus 17 carries out the following process for recovering methoxypropanols 24 from the aqueous stream 16. The aqueous stream 16 besides water contains 1-methoxypropanol, 2-methoxypropanol and remnant methanol.

Prior to passing the aqueous stream through the adsorption unit 28, methanol 18 is removed from the aqueous stream 16 by distillation, resulting in another aqueous stream 27, the bottoms stream of methanol stripping column 25, which is depleted by methanol. The recovered methanol 18 leaves the stripping column 25 overheads. In order to have a purified aqueous stream 27 that is depleted by practically all methanol 18, the distillation is carried out at a sump temperature that will also cause a certain amount of water leaving the stripping column 25 overheads. The methanol stream 18 thus may comprise a methanol/water mixture. The methanol/water mixture is partly fed back to the stripping column 25 as reflux and may be partly fed to the methanol separation section 11 of the plant for the production of propylene oxide. Thereby, additional methanol contained in the process water stream 16 is recovered and reused as solvent.

The aqueous stream 27 of the methanol stripping column 25, containing water, 1-methoxypropanol and 2-methoxypropanol, is then fed to the adsorption unit 28, In an adsorption mode of the adsorption unit 28, a purified aqueous stream 21 is obtained by passing the aqueous stream 27 through the adsorption unit 28 that contains an adsorption agent for adsorbing the methoxypropanols to the adsorption agent. The purified aqueous stream 21, depleted of methanol and methoxypropanols, can be subject to further treatment, e.g. a biological waste water treatment unit (not shown), forming a sink for the purified aqueous stream 21.

For recovering the methoxypropanols 24 adsorbed in the adsorption agent, the adsorption unit 28 is operated in a desorption mode, in which the methoxypropanols are desorbed from the adsorption agent. The step of desorbing the methoxypropanols comprises passing an alcohol as a desorption agent 30 through the adsorption unit 28 to desorb and discharge the methoxypropanols 24 from the adsorption agent.

To this end, desorption agent 30 is pumped from the buffer vessel 31 through the adsorption unit 28 and to the buffer vessel 34 for desorption agent loaded with methoxypropanols. From buffer vessel 34 the loaded desorption agent 48 is fed to the methoxypropanol/desorption agent separation column 35. Within separation column 35 the methoxypropanols 24 are separated from the desorption agent 30 by distillation. The desorption agent 30 depleted by methoxypropanols leaves the separation column 35 as bottoms stream back to buffer vessel 31. The methoxypropanols 24 are discharged overheads.

Preferably, a polyhydric alcohol, and even more preferred propylene glycol is used as the desorption agent. As already discussed before, propylene glycol can be supplied from the propylene glycol separation unit 15 (cf. Fig. 1) as a glycol make-up stream 19.

Preferably, the desorption agent 30 is passed through the adsorption unit 28 at a temperature of at least 60 °C, more preferably of at least 70 °C, in order to improve desorption efficiency.

Before the desorbing step is started, the adsorption unit 28 is preferably flushed with the desorption agent 30 to displace remaining water in the adsorption unit 28 resulting in a desorption agent/water-mixture 20 being a first flushing effluent 50. After the desorbing step the adsorption unit 28 is preferably flushed with the aqueous stream 27 to displace remaining desorption agent 30 in the adsorption unit 28 resulting in a desorption agent/water-mixture 20 being a second flushing effluent 51. Both flushing effluents 50, 51 may be collected in buffer vessel 33 and be returned via the connecting line for recycling flushing effluents 49 to the propylene glycol separation unit 14 (cf. Fig. 1).

The regeneration of the adsorption unit 28 is preferably controlled by control unit 43. The control unit 43 may switch between adsorption, desorption and flushing modes of the adsorption unit 28 by controlling the first and second switching means 41, 42, accordingly. The switching between the modes may be carried out based on continuous monitoring of the adsorption unit's 28 effluents, e.g. by means of sensors 53, 54, 55.

In particular, in the adsorption mode, the loading condition of the adsorption unit 28 can be detected by analysis of the stream outgoing from the adsorption unit 28 for breakthrough of methoxypropanols by means of the first sensor 53. Preferably, online analyzers are used for this task. Suitable analysis methods to be used are known to those skilled in the art.

In a preferred embodiment of the invention, the adsorption unit 28, or a section 28', 28" thereof, is considered as fully loaded, if a breakthrough of methoxypropanols in the outgoing water stream 21 is detected. In a furtherly preferred embodiment of the invention, the adsorption unit 28, or a section 28', 28" thereof, is considered as fully loaded if a certain threshold concentration of methoxypropanols in the outgoing purified aqueous stream 21 is reached. In other words, in these embodiments a predefined "slip amount" of methoxypropanols in the outgoing purified aqueous stream 21 is allowed.

After having detected the loading condition, the regeneration of the loaded adsorption unit 28, or a particular section 28', 28" thereof, is preferably executed as follows:

### Flushing with desorbing agent

From the loaded adsorption unit 28, or a section 28', 28" thereof, the still contained, remaining aqueous phase in the adsorption unit 28 is displaced by, preferably tempered, propylene glycol 30 from glycol buffer vessel 31. The resulting mixture of propylene glycol and water 20 is collected in the glycol/water buffering vessel 33 and from there pumped to the glycols recovery section 14 of the plant for the production of propylene oxide (cf. Fig. 1), where its glycol content is recovered from the water phase. During this flushing mode, the methoxypropanols contained in the glycol/water mixture remain in the glycol/water phase and subsequently are recycled to the adsorption unit 28 with the water stream 16.

The progress of displacement of water from the adsorption unit 28 is determined by analyzing the outgoing stream for water, e.g. by means of the second sensor 54. Preferably, online analyzers are used for this task. Suitable analysis methods are known to those skilled in the art.

### Desorption of loaded adsorption unit

When the water content in the flushing effluent 50 has been reduced to a predefined water threshold level, the flushing mode is terminated and the desorption mode is initiated.

Methoxypropanols are desorbed from the adsorption unit 28, or a particular section 28', 28" thereof, by further propylene glycol 30 and the desorption agent/methoxypropanols mixture is collected in the desorption agent/methoxypropanols buffer vessel 34 and from there fed to the desorption agent/methoxypropanols separation column 35. The progress of the desorption process is determined by analyzing the outflowing stream for methoxypropanols, e.g. by means of a third sensor 55. Preferably, online analyzers are used for this task. Suitable analysis methods are known to those skilled in the art.

In the desorption agent/methoxypropanols separation column 35, an azeotropic mixture of methoxypropanols and water is distilled overheads. The water content of this mixture is caused by the water content of the used desorption agent 30 on the one hand and a small water amount formed by thermal decomposition of the desorption agent during distillation on the other hand. The overheads stream 24 of column 35, containing 1-methoxypropanol, 2-methoxypropanol and water, is given to battery limits for further processing, preferably for combustion in an incineration unit and generating process steam.

The desorption agent separated at the column bottoms 35 is collected in buffer vessel 31 and re-used for displacement/desorption. Desorption agent from battery limits 19, preferably propylene glycol from the propylene glycol separation unit 14 of a plant for the production of propylene oxide, is used for make-up.

### Flushing with aqueous stream

After the regeneration/desorption phase, the desorption agent holdup within the adsorption unit 28 is displaced by a part of the bottoms stream 27 of the methanol stripping column 25. The outflowing desorption agent/water mixture is collected in desorption agent/water buffer vessel 33 and may be fed to the propylene glycol separation section 14 of a plant for the production of propylene oxide. The displacement of desorption agent from the adsorption unit 28 is monitored by analyzing the water content of the outflowing stream from the adsorption bed, e.g. by the second sensor 54. When the content of desorption agent is reduced to a predefined threshold level, the adsorption unit 28 is considered fully regenerated and ready for operation in adsorption mode, again.

In order to ensure a continuous treatment of the aqueous stream 27 in the adsorption unit 28, even inspite of the need of regular regeneration, the adsorption unit 28 shown in Fig. 2 is divided in two separately operable sections 28', 28", whereof at least temporarily a first section 28', 28" is operated in an adsorption mode, while a second section 28", 28' is operated in a desorption mode at the same time.

The sections 28', 28" may for example be operated independently in parallel with a shifted regeneration time schedule, such that at least one section 28', 28" is operating in adsorption mode at any time.

Another possibility is to operate the section 28', 28" in series. Then, the first section of the series will absorb the largest part of methoxypropanols. It will therefore become fully loaded first and will be switched to regeneration. During regeneration of the first section, the sections downstream of the first section will be operated in adsorption mode alone. After regeneration of the first section, all sections will be operated in series again, preferably with the first section being then connected downstream of the other sections, as the last section of the series.

In both cases, the time schedule of adsorption and desorption/regeneration should be set up in a way that assures continuous operation regarding the aqueous stream to be treated by adsorption of methoxypropanols.

The apparatus 17 may comprise also a system for heat recovery, in order to optimize energy consumption. In particular, the sensible heat of the bottoms stream 27 of the methanol stripping column 25 may be utilized for heating up the propylene glycol/methoxypropanol stream 48 from vessel 34 by heat exchange using a suitable heat exchanger. In addition or alternatively, the sensible heat of the bottoms stream of glycol/methoxypropanol separation column 35 may be utilized for heating up the propylene glycol/water stream 20 from vessel 33 by heat exchange using a suitable heat exchanger.

Fig. 3 shows a second embodiment of an apparatus 17 for recovering methoxypropanols 22, 23 from an aqueous stream 16, 27.

In comparison to the first embodiment shown in Fig. 2 the apparatus 17 shown in Fig. 3 differs by the fact that the mixed stream of methoxypropanols 24 being the overheads stream of column 35 is further treated in separation columns 36 and 38 for obtaining purified streams of 1-methoxypropanols 22 and 2-methoxypropanols 23.

To this end, the overheads stream of separation column 35 is fed to a 2-methoxypropanol separation column 36. In the 2-methoxypropanol separation column 36, the higher boiling 2-methoxypropanol 23 is separated as bottoms product. At the top of the separation column 36, a mixture of 1-methoxypropanol and water 37, is distilled off.

The water-containing 1-methoxypropanol 37 is fed to the 1-methoxypropanol-dewatering column 38. In this column, pure 1-methoxypropanol 22, is separated as bottoms product, while at the overhead of column 38, an azeotropic mixture of water and 1-methoxypropanol 37 is separated. In this embodiment of the invention, the separation columns 36 and 38 share a common overheads system. The separated and condensed azeotropic mixture of 1-methoxypropanol and water 37, is similarly used as reflux to column 36 as well as reflux and as feed to column 38. A part of the azeotropic 1-methoxypropanol/water mixture 39 may be recycled to the bottoms of methanol stripping column 25.

Alternatively (not shown), the 2-methoxypropanol separation column and the 1-methoxypropanol dewatering column are equipped with separate overhead condensing systems, comprising a condenser, a reflux vessel and a pump. In this embodiment, the product stream of the 2-methoxypropanol separation column is fed to the 1-methoxypropanol dewatering column and the product stream of 1-methoxypropanol dewatering column is recycled to the bottom of the methanol stripping column.

Regarding heat recovery, the embodiment of Fig. 3 may further be improved by utilizing the latent heat of the overheads stream 18 of the methanol stripping column 25 for heating of the reboilers of the 2-methoxypropanol separation column 36 and/or the 1-methoxypropanol dewatering column 38.

In all other respects, the above description of the first embodiment shown in Fig. 2 applies to the embodiment of Fig. 3, accordingly.

### List of Reference Signs

- 1: Fresh propylene
- 2: Aqueous hydrogen peroxide solution
- 3: Reactor section
- 4: Reaction mixture
- 5: Propylene separation unit
- 6: Recycle propylene
- 7: Reaction mixture depleted by propylene
- 8: Propylene oxide separation unit
- 9: Reaction mixture depleted by propylene oxide
- 10: Purified propylene oxide
- 11: Methanol separation unit
- 12: Methanol to be recycled
- 13: Reaction mixture depleted by methanol
- 14: Propylene glycol separation unit
- 15: Purified propylene glycol
- 16: Aqueous stream of process water from propylene oxide plant
- 17: Apparatus for recovering methoxypropanols from an aqueous stream
- 18: Removed methanol (diluted with water)
- 19: Glycol make-up stream
- 20: Desorption agent/water mixture
- 21: Purified aqueous stream
- 22: Purified 1-methoxypropanol
- 23: Purified 2-methoxypropanol
- 24: Mixed methoxypropanols
- 25: Methanol stripping column
- 26: 2-methoxypropanol separation column
- 27: Bottoms stream of methanol stripping column, depleted by methanol
- 28: Adsorption unit
- 28', 28": Separately operable sections
- 30: Desorption agent
- 31: Buffer vessel for desorption agent
- 33: Buffer vessel for flushing effluents
- 34: Buffer vessel for desorption agent loaded with methoxypropanols
- 35: Methoxypropanol/desorption agent separation column
- 36: 2-methoxypropanol separation column
- 37: 1-methoxypropanol/water mixture
- 38: 1-methoxypropanol dewatering column
- 39: 1-methoxypropanol /water recycle stream
- 40: Mixed stream
- 41: First switching means
- 42: Second switching means
- 43: Control unit
- 44: Inlet
- 45: Outlet
- 46: Valves of second switching means
- 47: Valves of first switching means
- 48: Desorption agent loaded with methoxypropanols
- 49: Connecting line for recycling flushing effluents
- 50: First flushing effluent
- 51: Second flushing effluent
- 52: Separation section
- 53, 54, 55: Sensors

## Claims

1. Process for recovering methoxypropanols (22, 23, 24) from an aqueous stream (16, 27), wherein the process comprises the steps of:
obtaining a purified aqueous stream (21) by passing the aqueous stream (16, 27) through an adsorption unit (28) containing an adsorption agent for adsorbing the methoxypropanols to the adsorption agent,
desorbing the methoxypropanols from the adsorption agent,
**characterized in that** the step of desorbing the methoxypropanols comprises
passing an alcohol as a desorption agent (30) through the adsorption unit (28) to desorb and discharge the methoxypropanols (22, 23, 24) from the adsorption agent,
separating the methoxypropanols (22, 23, 24) from the desorption agent (30) by distillation.

2. Process according to claim 1, **characterized in that** a polyhydric alcohol, preferably propylene glycol is used as the desorption agent (30).

3. Process according to claim 1 or 2, **characterized in that** the desorption agent (30) is passed through the adsorption unit (28) at a temperature of at least 60 °C, preferably at least 70 °C.

4. Process according to any one of the claims 1 to 3, **characterized in that** prior to passing the aqueous stream (27) through the adsorption unit (28), methanol (18) is removed from the aqueous stream (16) by distillation.

5. Process according to any one of the claims 1 to 4, **characterized in that** before the desorbing step is started, the adsorption unit (28) is flushed with the desorption agent (30) to displace remaining water in the adsorption unit (28) resulting in a desorption agent/water-mixture (20) forming a first flushing effluent (50).

6. Process according to any one of the claims 1 to 5, **characterized in that** after the desorbing step the adsorption unit (28) is flushed with the aqueous stream (16, 27) to displace remaining desorption agent (30) in the adsorption unit (28) resulting in a desorption agent/water-mixture (20) forming a second flushing effluent (51).

7. Process according to any one of the claims 1 to 6, **characterized in that** the adsorption unit (28) is divided in at least two separately operable sections (28', 28"), whereof at least temporarily a first section (28') is operated in an adsorption mode, while a second section (28") is operated in a desorption mode at the same time.

8. Process for producing of propylene oxide (10), comprising the steps of
catalytically oxidizing propylene (1, 6) with an aqueous hydrogen peroxide solution (2) using methanol (12) as solvent thereby obtaining a reaction mixture (4),
separating from the reaction mixture (4)
unreacted propylene (6) in a propylene separation unit (5) to be recycled, propylene oxide (10) as main reaction product in a propylene oxide separation unit (8),
methanol (12) as the solvent in a methanol separation unit (11) to be recycled, and propylene glycol (15) as a reaction by-product in a propylene glycol separation unit (14),
leaving a remaining aqueous stream containing methoxypropanols (16),
**characterized in that** methoxypropanols (22, 23, 24) are recovered from the aqueous stream (16) using the process according to any one of the claims 1 to 7.

9. Process according to claim 8, **characterized in that** the propylene glycol (15) obtained as reaction by-product is used as the desorption agent (30).

10. Process according to claim 9 and claim 5 and 6, **characterized in that** the first flushing effluent (51) and/or the second flushing effluent (52) is recycled to the propylene glycol separation unit (14).

11. Apparatus for recovering methoxypropanols (22, 23, 24) from an aqueous stream (16, 27), comprising:
an adsorption unit (28) comprising an adsorption agent for adsorbing the methoxypropanols and having an inlet (44) and an outlet (45),
**characterized in that** the apparatus (17) further comprises
a buffer vessel (31) for storing a desorption agent (30),
a distillation unit (35) for separating the methoxypropanols (22, 23, 24) from the desorption agent (30), and
first and second switching means (41, 42),
wherein the buffer vessel (31) and a source of the aqueous stream (16, 27) are connected to the inlet (44) via the first switching means (41) and the distillation unit (35) and a sink for a purified aqueous stream (21) are connected to the outlet via the second switching means (42), for alternately passing the aqueous stream (16, 27) and the desorption agent (30) through the adsorption unit (28) to switch over between in an adsorption mode and a desorption mode of the adsorption unit (28),
wherein the distillation unit (35) is connected to the buffer vessel (31) for recycling the desorption agent (30).

12. Apparatus according to claim 11, **characterized in that** between the source of the aqueous stream (16) and the first switching means (41) a methanol stripping column (25) is arranged in order to remove methanol (18) from the aqueous stream (16) by distillation.

13. Plant for producing of propylene oxide (10), comprising
a reactor section (3) for catalytically oxidizing propylene (1, 6) with an aqueous hydrogen peroxide solution (2) using methanol (12) as solvent thereby obtaining a reaction mixture (4),
and downstream of the reactor section (3) a separation section (52) for obtaining an aqueous stream (16, 27) from the reaction mixture (4), the separation section (52) comprising
a propylene separation unit (5) for separating unreacted propylene (6) from the reaction mixture (4) to be recycled to the reactor section (3),
a propylene oxide separation unit (8) for separating propylene oxide (10) from the reaction mixture (4) as main reaction product,
a methanol separation unit (11) for separating methanol (12) from the reaction mixture (4) to be recycled to the reactor section (3), and
a propylene glycol separation unit (14) for separating propylene glycol (15) from the reaction mixture (4) as a reaction by-product,
**characterized in that** the plant comprises downstream of the separation section (52) an apparatus (17) for recovering methoxypropanols according to claim 11 or 12 from the aqueous stream (16) obtained by the separation section (52).

14. Plant for producing of propylene oxide according to claim 13, **characterized in that** the propylene glycol separation unit (14) is connected to the buffer vessel (31) in order to provide the reaction by-product propylene glycol (19) as the desorption agent (30).

15. Plant for producing of propylene oxide according to claim 13 or 14, **characterized in that** the second switching means (42) is connected to the propylene glycol separation unit (14) by means of a connecting line for recycling flushing effluents (49) obtained in a flushing mode of the adsorption unit (28) to the propylene glycol separation unit (14).

16. Plant for producing of propylene oxide according to any one of the claims 13 to 15, **characterized in that** the adsorption unit (28) contains at least two sections (28', 28"), that are connected to the first (41) and second switching means (42) in such a way, that a first section (28') can be operated in an adsorption mode, while a second section (28") is operated in a desorption mode at the same time.
